Europäisches Patentamt

European Patent Office

Office européen des brevets

(11) Numéro de publication : **0 038 730**
**B1**

# (12) FASCICULE DE BREVET EUROPÉEN

(45) Date de publication du fascicule du brevet :
**26.09.84**

(51) Int. Cl.³ : **C 08 F 12/02**, C 08 F 2/44

(21) Numéro de dépôt : **81400519.5**

(22) Date de dépôt : **01.04.81**

(54) **Latex de polymères magnétiques et procédé de préparation.**

(30) Priorité : **18.04.80 FR 8008696**

(43) Date de publication de la demande :
**28.10.81 Bulletin 81/43**

(45) Mention de la délivrance du brevet :
**26.09.84 Bulletin 84/39**

(84) Etats contractants désignés :
**AT BE CH DE FR GB IT LI LU NL SE**

(56) Documents cités :
**FR-A- 2 167 992**
**FR-A- 2 334 106**
**FR-A- 2 463 778**
**GB-A- 1 416 004**
**JP-A-52 054 311**
**US-A- 3 725 285**
**US-A- 4 157 323**
**CHEMICAL ABSTRACTS, vol. 85, no. 12, 20 septembre 1976, page 45 colonne 1, abrégé 78974v Columbus, Ohio, US**
**CHEMICAL ABSTRACTS, vol. 85, no. 10, 06 septembre 1976, page 28, 2ème colonne, abrégé 63703g Columbus, Ohio, US**
**Le dossier contient des informations techniques présentées postérieurement au dépôt de la demande et ne figurant pas dans le présent fascicule.**

(73) Titulaire : **RHONE-POULENC SPECIALITES CHIMIQUES**
**"Les Miroirs" 18, Avenue d'Alsace**
**F-92400 Courbevoie (FR)**

(72) Inventeur : **Daniel, Jean-Claude**
**13, rue de Neuilly**
**F-94120 Fontenay-sous-Bois (FR)**
Inventeur : **Schuppiser, Jean-Luc**
**12, Allée d'Apollon Gressy**
**F-77410 Claye-Souilly (FR)**
Inventeur : **Tricot, Marc**
**décédé (FR)**

(74) Mandataire : **Fabre, Madeleine-France et al**
**RHONE-POULENC RECHERCHES Service Brevets Chimie et Polymères 25, quai Paul Doumer**
**F-92408 Courbevoie Cedex (FR)**

## Description

L'invention a pour objet des latex de polymères vinylaromatiques hydrophobes contenant une charge magnétique, ainsi qu'un procédé de préparation desdits latex.

Des polymères magnétiques sous forme de gels ou de particules, obtenus par mélange de la charge magnétique aux polymères préparés par les techniques classiques de polymérisation en masse, solution, émulsion ou suspension, suivi d'opérations comme par exemple atomisation, coagulation, extrusion ou distillation de solvant, sont décrits notamment dans les brevets français n° 2 334 106 et n° 2 167 992 et le brevet anglais n° 1 416 004. Il s'agit de produits constitués de particules de forme plus ou moins régulière comportant des défauts de répartition de la charge magnétique ; en outre le fait de réaliser un mélange préalable constitue une étape supplémentaire nécessitant un appareillage approprié et une consommation non négligeable d'énergie et présente des difficultés de dispersion de la charge magnétique dans les polymères.

Le mélange de la charge magnétique à un latex de polymère est également connu (brevet américain n° 3 725 285 et demande japonaise Kokai n° 52-54311), mais il est nécessaire d'avoir des polymères particuliers pour obtenir la dispersion de la charge magnétique et les produits obtenus présentent une stabilité souvent insuffisante.

Des latex de polymères hydrophiles magnétiques contenant des particules de polymères hydrophiles de diamètre inférieur à 2 μm renfermant une charge magnétique font l'objet du brevet américain n° 4 157 323. Ils sont obtenus en dispersant une charge magnétique dans une solution aqueuse d'une composition monomère constituée d'un monomère totalement hydrophile, d'un monomère faiblement soluble dans l'eau comportant des caractères hydrophobes mais non totalement hydrophobes et d'un agent réticulant diénique ou triénique soluble dans l'eau, puis en polymérisant en émulsion. De tels latex magnétiques sont hydrophiles et d'applications limitées.

Quant aux monomères vinylaromatiques totalement hydrophobes, leur polymérisation en émulsion en présence de la charge magnétique ne donne pas de résultats satisfaisants, car au cours de la polymérisation il n'y a pas incorporation de la charge magnétique dans les particules de polymères.

Les produits de l'invention ne présentent pas ces inconvénients, ils sont stables, avec une bonne dispersion de la charge magnétique dans les particules de polymères vinylaromatiques hydrophobes et sont obtenus facilement par polymérisation de monomères vinylaromatiques insolubles dans l'eau en présence de la charge magnétique.

Les latex de polymères magnétiques selon l'invention ont une concentration de polymères de diamètre moyen compris entre 0,03 et 5 μm, inférieure à 65 % en poids et sont caractérisés en ce que les particules sont constituées de polymères vinylaromatiques hydrophobes et renferment 0,5 à 50 % en poids d'une charge magnétique par rapport au polymère.

Par polymères vinylaromatiques hydrophobes, on entend les homopolymères de monomères vinylaromatiques insolubles dans l'eau, tels que styrène, alpha-méthylstyrène, éthylstyrène, tertio-butylstyrène, vinyltoluène, ainsi que les copolymères de ces monomères entre eux et/ou avec d'autres monomères copoly·ıérisables insolubles dans l'eau, choisis parmi les composés diéniques, les acrylates et méthacrylates d'alkyle dont le groupe alkyle possède 3 à 10 atomes de carbone, les esters d'acides éthyléniques possédant 4 ou 5 atomes de carbone et d'alkyle possédant 1 à 8 atomes de carbone.

Dans les particules, la charge magnétique représente 0,5 à 50 %, plus particulièrement 0,5 à 35 % et de préférence 0,7 à 20 % en poids par rapport au polymère.

Dans le latex, la concentration en particules de polymères est inférieure à 65 % et de préférence comprise entre 5 et 50 % en poids. Toutefois le latex peut-être dilué ou concentré sans inconvénient. Les particules ont un diamètre moyen compris entre 0,03 à 5 μm et de préférence entre 0,05 et 1 μm. Ces particules possèdent une répartition granulométrique large ou étroite, suivant les conditions de polymérisation mises en œuvre et sont attirées par un aimant, même lorsque le latex est très dilué, par exemple lorsque la concentration est aussi basse que 1 % en poids.

Le procédé d'obtention des latex de polymères magnétiques consiste à polymériser en milieu aqueux un monomère seul ou en mélange avec un monomère copolymérisable en présence d'une charge magnétique et est caractérisé en ce que la charge magnétique est dispersée dans une phase organique formée d'un initiateur organosoluble, de tout ou partie d'un monomère vinylaromatique seul ou en mélange avec au moins un monomère copolymérisable, donnant un polymère hydrophobe et/ou d'un composé organique insoluble dans l'eau ; la dispersion obtenue est mélangée avec tout ou partie d'une solution aqueuse formée d'eau et d'au moins un agent émulsifiant, puis l'ensemble est homogénéisé et enfin polymérisé après addition éventuelle du reste des constituants.

Suivant le procédé de l'invention, et selon les composés mis en œuvre, la phase organique dans laquelle la charge magnétique est dispersée est constituée par :
— tout ou partie du ou des monomères et la totalité de l'initiateur ;
— ou bien la totalité du composé organique insoluble dans l'eau et la totalité de l'initiateur ;
— ou encore la totalité du composé organique, tout ou partie du ou des monomères, et la totalité de l'initiateur.

Dans les cas où le ou les monomères ne sont pas présents en partie ou en totalité dans la phase organique, ladite phase organique, ne contenant qu'une partie du ou des monomères ou ne contenant pas de monomères, peut représenter au minimum 1 % de la totalité de la phase organique. Les monomères sont alors ajoutés au milieu soit après homogénéisation et avant polymérisation, soit en cours de polymérisation par fractions successives ou en continu, soit en partie avant polymérisation, en partie en cours de polymérisation par fractions successives ou en continu.

Les monomères à mettre en œuvre dans le procédé doivent donner des polymères hydrophobes. Ils sont insolubles dans l'eau et représentés par les composés vinylaromatiques, tels que : styrène, alpha-méthylstyrène, éthylstyrène, tertio-butylstyrène, vinyltoluène.

Ces monomères sont utilisés seuls ou en mélange entre eux en toute proportion, ou encore en mélange avec un autre monomère copolymérisable, insoluble dans l'eau, pouvant constituer jusqu'à 50 % du mélange et choisi parmi les composés diéniques, tels que butadiène, isoprène ; les acrylates et méthacrylates d'alkyle dont le groupe alkyle possède 3 à 10 atomes de carbone ; les esters d'acides éthyléniques possédant 4 ou 5 atomes de carbone et d'alkyle possédant 1 à 8 atomes de carbone, tels que : furmarate d'heptyle, fumarate d'octyle, itaconate de méthyle, itaconate d'éthyle.

Suivant le polymère à obtenir, il est possible d'ajouter au monomère ou au mélange de monomères, un monomère réticulant et/ou un agent limiteur de chaîne. Le monomère réticulant, mis en œuvre en proportions comprises entre 0 et 5 % en poids par rapport au(x) monomère(s), est représenté plus particulièrement par le divinylbenzène, le méthacrylate de vinyle, les acrylates ou méthacrylates de mono- ou polyalkylène (2-4 C) glycol, le cyanurate de triallyle, les condensats d'acides carboxyliques insaturés et de polyols, comme par exemple les acrylate et méthacrylate de triméthylolpropane. Quant à l'agent limiteur de chaîne, ses proportions sont de 0 à 5 % en poids par rapport au(x) monomère(s) et il est représenté notamment par le dimère de l'alpha-méthylstyrène, les alkylmercaptans à chaîne linéaire ou ramifiée, les hydrocarbures halogénés.

Le composé organique insoluble dans l'eau doit être miscible avec le ou les monomères et éventuellement avec l'initiateur et ne doit pas inhiber la polymérisation. Il est choisi plus particulièrement parmi : les hydrocarbures aliphatiques saturés et non saturés, alicycliques et aromatiques, halogénés ou non, ayant de 10 à 30 atomes de carbone, tels que : dodécane, hexadécane, heptadécane, eicosane, cires paraffiniques, paraffines chlorées, décène-1, do-, tétra-, hexa-, hepta-, octa-décène1, eicosène-1, tétra-, hexa-éthylbenzène, naphtalène, anthracène ; les alcools aliphatiques saturés ou non saturés possédant de 10 à 30 atomes de carbone, tels que : alcools laurique, myristique, cétylique, stéarylique, eicosylique, oléique ; les esters d'acide minéraux ou organiques ayant de 1 à 20 atomes de carbone et d'alcools ayant de 1 à 20 atomes de carbone, dont le nombre d'atomes de carbone est d'au moins 10, tels que : phosphate de tricrésyle, formates de cétyle, de stéaryle, adipate de dioctyle, sébacate de dibutyle, laurates de propyle, d'éthylhexyle, palmitate d'éthyle, phtalates de dioctyle, de diéthylhexyle, de dicyclohexyle, de dibenzyle ; les polymères de bas poids moléculaire, tels que : polybutadiènes liquides, cires et huiles de polyéthylène. La quantité de composé organique à mettre en œuvre peut représenter jusqu'à 50 % et de préférence jusqu'à 20 % en poids de la totalité de la phase organique contenant la charge magnétique.

Les initiateurs mis en œuvre seuls ou en mélange, en quantité comprise entre 0,1 et 10 % en poids par rapport au(x) monomère(s), sont choisis parmi les initiateurs organosolubles de polymérisation classiques. Ce sont, par exemple, les azonitriles, comme l'azobisisobutyronitrile, l'azobiscyclohexane carbonitrile ; ou les peroxydes, comme les peroxydes de benzoyle, dicumyle, ditertiobutyle, diacétyle, dioctanoyle, lauroyle, méthyléthylcétone, caprylyle, dichloro-2,4-benzoyle, parachlorobenzoyle ; les perpivalate, diéthylperacétate, perbenzoate de tertiobutyle ; le diperphtalate de ditertiobutyle ; le 1,1-ditertiobutyle-peroxyde-3,3,5-triméthylcyclohexane.

L'initiateur doit être dissous dans la phase organique, avant homogénéisation. Dans certains cas, afin de faciliter sa dissolution dans la phase organique, il peut être avantageux de dissoudre préalablement l'initiateur dans une petite quantité d'un solvant organique miscible avec la phase organique et non inhibiteur de la polymérisation, comme par exemple un hydrocarbure aliphatique ou aromatique ayant moins de 10 atomes de carbone et éventuellement halogéné, tel que : chlorure de méthylène, chloroforme, tétrachlorure de carbone, dichloréthane, benzène ou chlorobenzène.

La charge magnétique, qui est dispersée dans la phase organique, se présente sous forme de particules suffisamment fines pour pouvoir être incluses dans les particules de polymère du latex à obtenir, c'est-à-dire que leur taille est généralement inférieure à 1 μm et de préférence comprise entre 0,002 et 0,05 μm. Elle est représentée plus particulièrement par :

— des métaux, ou leurs alliages, tels que ; fer, fer-silicium, nickel, cobalt, ou les alliages avec du molybdène, du chrome, du cuivre, du vanadium, du manganèse, de l'aluminium, du titane ;

— des oxydes de fer ; $Fe_3O_4$ ou $\gamma\text{-}Fe_2O_3$ pur ou en combinaison ou en mélange avec d'autres oxydes comme les oxydes de cobalt, de manganèse, de zinc, de baryum, de terres rares ;

— du dioxyde de chrome.

La quantité de charge magnétique à mettre en œuvre est comprise entre 0,5 et 50 %, plus particulièrement entre 0,5 et 35 % et de préférence entre 0,7 et 20 % en poids par rapport au(x) monomère(s). Avant incorporation à la phase organique, la charge magnétique est avantageusement dispersée dans tout ou partie du composé organique, et/ou d'un solvant organique choisi parmi les solvants mentionnés plus haut pour l'initiateur.

La phase aqueuse à mettre en œuvre est constituée par de l'eau, dans laquelle sont dissous au moins

3

un agent émulsifiant et le cas échéant un tampon, suivant la nature du monomère et/ou de l'émulsifiant présents.

Les agents émulsifiants, qui assurent la stabilité du milieu réactionnel et du latex à obtenir, peuvent être anioniques, ou cationiques, et/ou non ioniques et sont utilisés dans des quantités comprises entre 0,1 et 5 % en poids par rapport à la phase organique contenant la charge magnétique.

Parmi les agents émulsifiants anioniques peuvent être cités les sels d'acides gras ; les alkylsulfates, alkylsulfonates, alkylarylsulfonates, alkylsulfosuccinates, alkylphosphates alcalins ; les sulfosuccinates d'alkyle ; les sulfonates d'éthers alkylphénolpolyglycoliques ; les sels d'esters d'acides alkylsulfopolycarboxyliques ; les produits de condensation des acides gras avec les acides oxy- et amino-alcanesulfoniques ; les dérivés sulfatés des éthers polyglycoliques ; les esters sulfatés d'acides gras et de polyglycols ; les alcanolamides d'acides gras sulfatés.

Parmi les agents émulsifiants cationiques peuvent être cités les alkylamines et leurs sels solubles dans l'eau, les sels solubles des alkylamines N-substituées par des radicaux alkyle et/ou alkylaryle et/ou hydroxyalkyle. Et, parmi les agents émulsifiants non ioniques, les esters gras de polyalcools, les alcanolamides d'acides gras, les polyoxydes d'éthylène, les copolyoxydes d'éthylène et de propylène, les alkylphénols oxyéthylénés.

Lorsque la totalité de la phase aqueuse ne participe pas à l'opération d'homogénéisation, la quantité minimum de phase aqueuse à mettre en œuvre doit être telle que le rapport phase organique/phase aqueuse soit de l'ordre de 1. La partie de phase aqueuse restante est alors introduite dans le milieu réactionnel avant et/ou en cours de polymérisation, par fractions successives ou en continu.

Après préparation de la phase organique contenant la charge magnétique et de la phase aqueuse, ces deux phases sont mélangées pour obtenir la dispersion à homogénéiser. Pour ces opérations, la phase organique doit être liquide et homogène. Dans certains cas, il est donc nécessaire d'opérer à une température suffisante pour avoir la phase organique liquide, ou bien d'ajouter un solvant de la phase organique, insoluble dans l'eau et non inhibiteur de polymérisation, en quantité juste nécessaire pour obtenir une phase liquide homogène. Le solvant mis en œuvre est choisi parmi les solvants mentionnés plus haut.

L'opération d'homogénéisation est effectuée sur la dispersion de la phase organique contenant la charge magnétique dans tout ou partie de la phase aqueuse à une température comprise entre 0 °C et une température inférieure à la température de décomposition de l'initiateur, à l'aide d'un moyen mécanique énergique, tel que, et sans que la liste soit limitative, moulin à colloïdes, pompe rapide, agitateur à vibrations, appareils à ultra-sons, afin d'obtenir des gouttelettes de phase organique contenant la charge magnétique de taille comprise entre 0,03 et 5 μm.

Le mélange finement dispersé obtenu, auquel on a éventuellement ajouté le ou les monomères restant et une partie de la phase aqueuse, est ensuite polymérisé en microsuspension de façon en soi connue à des températures comprises entre 30 et 130 °C et avec une concentration de la phase organique contenant la charge magnétique, dans le milieu réactionnel inférieure à 65 % en poids.

Les latex de polymères obtenus possèdent des particules de polymères de diamètre compris entre 0,03 et 5 μm et de préférence entre 0,05 et 1 μm, contenant la charge magnétique.

Les particules sont facilement attirées par un aimant, même lorsque la concentration est très faible, ce qui permet, dans certaines applications, de les séparer du milieu liquide ambiant, sans avoir recours aux procédés classiques de coagulation et atomisation.

Les latex selon l'invention peuvent être utilisés dans les industries des peintures, bandes magnétiques, enregistrement, en biologie, entre autres comme supports de molécules biologiques.

On donne ci-après, à titre indicatif et non limitatif, des exemples de réalisation de l'invention.

Exemple 1

Une phase organique est préparée par dissolution de 4 g de peroxyde de lauroyle dans 60 g de styrène, solution à laquelle sont ajoutés 5 g d'une dispersion à 16 % en poids dans une huile hydrocarbonée de $Fe_3O_4$, dont la taille des particules est de 0,01 μm environ.

Dans un récipient muni d'un agitateur, une phase aqueuse est préparée par dissolution, dans 400 g d'eau déminéralisée, de 1 g de laurylsulfate de sodium et 1 g de nonylphénol oxyéthyléné (30 motifs d'oxyde d'éthylène par molécule).

La phase organique est alors ajoutée et dispersée dans la phase aqueuse.

Le mélange obtenu est ensuite homogénéisé à température ambiante de façon à obtenir des gouttelettes de phase organique contenant la charge magnétique, dispersées dans la phase aqueuse, de 1 μm.

Le mélange est introduit dans un réacteur de 1 litre muni d'un agitateur, où il est polymérisé à 55 °C.

Après 19 heures, le milieu réactionnel est refroidi et le monomère résiduel éliminé par entraînement à la vapeur d'eau.

On obtient 500 g de latex stable, dont la concentration en particules de polystyrène est de 9,6 % en poids et les particules de couleur marron clair ont un diamètre moyen de 1 μm. La microscopie électronique montre que la charge magnétique qui représente 1,6 % en poids du polymère se trouve sous forme d'inclusions à la périphérie des particules.

Les particules du latex sont facilement séparées du milieu par attraction le long des parois d'un récipient au moyen d'un aimant. Le même phénomène est constaté si le latex obtenu est dilué à une concentration de 1 % en poids.

Exemple 2

La phase organique est constituée de :

— 5,45 g d'hexadécane,
— 0,80 g de peroxyde de lauroyle dissous dans 2,9 g de chlorobenzène,
— 5,45 g de la même dispersion de $Fe_3O_4$ que celle de l'exemple 1.

La phase aqueuse est constituée de :

—600   g d'eau déminéralisée,
—   1,6 g de laurylsulfate de sodium,
—   1,6 g de nonylphénol oxyéthyléné (30 motifs d'oxyde d'éthylène par molécule).

Comme dans l'exemple 1, phases organique et aqueuse sont mélangées et homogénéisées de façon à obtenir des gouttelettes de phase organique contenant la charge magnétique, dispersées dans la phase aqueuse, de 0,1 μm.

Le mélange est introduit dans un réacteur de 4 litres muni d'un agitateur, où il est chauffé à 63 °C, température qui est maintenue. Dès que le mélange est à 63 °C, 110 g de styrène distillé sont introduits, à débit constant pendant 10 heures.

Le mélange est laissé à 63 °C encore pendant 10 heures, puis chauffé à 70 °C pendant 5 heures. Le milieu réactionnel est alors refroidi.

730 g de latex stable sont obtenus, dont la concentration en particules de polymère est de 17 % en poids. Les particules de couleur marron clair ont un diamètre moyen de 0,15 μm et portent, incluses à leur périphérie, des particules de charge magnétique représentant 0,8 % en poids du polymère. Elles sont attirées par un aimant.

Exemple 3

La phase organique est constituée de :

— 5 g d'hexadécane,
— 4 g de peroxyde de lauroyle dissous dans 100 g de styrène,
— 5 g de la même dispersion de $Fe_3O_4$ que celle de l'exemple 1.

La phase aqueuse est formée de :

—400 g d'eau déminéralisée,
—   1 g de laurylsulfate de sodium,
—   1 g de nonylphénol oxyéthyléné (30 motifs d'oxyde d'éthylène par molécule).

Comme dans l'exemple 1, phases organique et aqueuse sont mélangées et homogénéisées de façon à obtenir des gouttelettes de phase organique contenant la charge magnétique d'un diamètre moyen de 1 μm, dispersées dans la phase aqueuse.

La dispersion, introduite dans un réacteur de 4 litres muni d'un agitateur, est chauffée à 60 °C et maintenue à cette température pendant 18 heures.

Après refroidissement du milieu réactionnel, 510 g de latex stable sont obtenus, dont la concentration en particules de polystyrène est de 17 % en poids.

Les particules de couleur marron clair ont un diamètre moyen de 1 μm et contiennent, réparties à leur périphérie, 1,1 % en poids du polymère de particules de charge magnétique. Elles sont attirées par un aimant.

Exemple 4

La phase organique est constituée de :

— 8,6 g d'hexadécane,
— 6,9 g de peroxyde de lauroyle dissous dans 172,5 g de vinyltoluène,
— 8,6 g de la même dispersion de $Fe_3O_4$ que celle de l'exemple 1.

5

La phase aqueuse est formée de :

— 690  g d'eau déminéralisée,
—   1,7 g de laurylsulfate de sodium,
—   1,7 g de nonylphénol oxyéthyléné (30 motifs d'oxyde d'éthylène par molécule).

Comme dans l'exemple 1, phases organique et aqueuse sont mélangées et homogénéisées de façon à obtenir des gouttelettes de phase organique contenant la charge magnétique d'un diamètre moyen de 0,16 μm, dispersées dans la phase aqueuse.

La dispersion, introduite dans un réacteur de 4 litres muni d'un agitateur, est chauffée à 60 °C pendant 18 heures, puis à 75 °C pendant 2 heures.

Après refroidissement du milieu réactionnel, 860 g de latex stable sont obtenus, dont la concentration en particules de polyvinyltoluène est de 19,7 % en poids.

Les particules de couleur marron clair ont un diamètre moyen de 0,16 μm et contiennent, réparties à leur périphérie, 0,8 % en poids du polymère de particules de charge magnétique. Elles sont attirées par un aimant.

Exemple 5

La phase organique est constituée de :

— 8,5 g d'hexadécane,
— 6,8 g de peroxyde de lauroyle dissous dans un mélange formé de 153,5 g de styrène et de 17 g d'acrylate de butyle,
— 8,5 g de la même dispersion de $Fe_3O_4$ que celle de l'exemple 1.

La phase aqueuse est formée de :

— 683  g d'eau déminéralisée,
—   1,7 g de laurylsulfate de sodium,
—   1,7 g de nonylphénol oxyéthyléné (30 motifs d'oxyde d'éthylène par molécule).

Comme dans l'exemple 1, phases organique et aqueuse sont mélangées et homogénéisées de façon à obtenir des gouttelettes de phase organique contenant la charge magnétique d'un diamètre moyen de 0,16 μm, dispersées dans la phase aqueuse.

La dispersion, introduite dans un réacteur de 4 litres muni d'un agitateur, est chauffée à 60 °C pendant 18 heures, puis à 75 °C pendant 2 heures.

Après refroidissement du milieu réactionnel, 860 g de latex stable sont obtenus, dont la concentration en particules de copolymère styrène-acrylate de butyle est de 19,9 % en poids.

Les particules de couleur marron clair ont un diamètre moyen de 0,16 μm et contiennent, réparties à leur périphérie, 0,8 % en poids du polymère de particules de charge magnétique. Elles sont attirées par un aimant.

Exemple 6

La phase organique est constituée de :

— 7,5 g d'alcool cétylique,
— 6  g de peroxyde de lauroyle dissous dans 151 g de styrène,
— 7,5 g de la même dispersion de $Fe_3O_4$ que celle de l'exemple 1.

La phase aqueuse est formée de :

— 605  g d'eau déminéralisée,
—   1,5 g de laurylsulfate de sodium,
—   1,5 g de nonylphénol oxyéthyléné (30 motifs d'oxyde d'éthylène par molécule).

Comme dans l'exemple 1, phases organique et aqueuse sont mélangées et homogénéisées de façon à obtenir des gouttelettes de phase organique contenant la charge magnétique d'un diamètre moyen de 0,15 μm, dispersées dans la phase aqueuse.

La dispersion, introduite dans un réacteur de 4 litres muni d'un agitateur, est chauffée à 60 °C pendant 18 heures, puis à 75 °C pendant 2 heures.

Après refroidissement du milieu réactionnel, 755 g de latex stable sont obtenus, dont la concentration en particules de polystyrène est de 19 % en poids.

Les particules de couleur marron clair ont un diamètre moyen de 0,15 µm et contiennent, réparties à leur périphérie, 0,8 % en poids du polymère de particules de charge magnétique. Elles sont attirées par un aimant.

**Revendications** (pour les Etats contractants : BE, CH, DE, FR, GB, IT, LI, LU, NL, SE)

1. Latex de polymères magnétiques de concentration en particules de polymères de diamètre moyen compris entre 0,03 et 5 µm, inférieure à 65 % en poids, caractérisés en ce que les particules sont constituées de polymères vinylaromatiques hydrophobes et renferment 0,5 à 50 % en poids d'une charge magnétique par rapport au polymère.

2. Latex selon la revendication 1, caractérisés en ce que les polymères vinylaromatiques sont représentés par les homopolymères de monomères vinylaromatiques insolubles dans l'eau, tels que styrène, alpha-méthylstyrène, éthylstyrène, tertiobutylstyrène, vinyltoluène ; et les copolymères de ces monomères entre eux et/ou avec d'autres monomères copolymérisables, insolubles dans l'eau, choisis parmi les composés diéniques, les acrylates et méthacrylates d'alkyle présentant un groupe alkyle en $C_3$-$C_{10}$, les esters alkyliques d'acides éthyléniques en $C_4$-$C_5$ présentant un groupe alkyle en $C_1$-$C_8$.

3. Procédé de préparation des latex selon la revendication 1 qui consiste à polymériser en milieu aqueux un monomère seul ou en mélange avec un monomère copolymérisable en présence d'une charge magnétique et est caractérisé en ce que la charge magnétique est dispersée dans une phase organique formée d'un initiateur organosoluble, de tout ou partie d'un monomère vinylaromatique seul ou en mélange avec au moins un monomère copolymérisable, donnant un polymère hydrophobe et/ou d'un composé organique insoluble dans l'eau ; la dispersion obtenue est mélangée avec tout ou partie d'une solution aqueuse formée d'eau et d'au moins un agent émulsifiant, puis l'ensemble est homogénéisé pour obtenir des gouttelettes de phase organique de taille comprise entre 0,03 et 5 µm et enfin polymérisé après addition éventuelle du reste des constituants.

4. Procédé selon la revendication 3, caractérisé en ce que les monomères vinylaromatiques mis en œuvre seuls ou en mélange entre eux, sont insolubles dans l'eau et sont représentés par le styrène, l'alpha-méthylstyrène, l'éthylstyrène, le tertiobutylstyrène, le vinyltoluène.

5. Procédé selon la revendication 3, caractérisé en ce que les monomères copolumérisables sont insolubles dans l'eau et sont choisis parmi les composés diéniques, les acrylates et méthacrylates d'alkyle présentant un groupe alkyle en $C_3$-$C_{10}$, les esters alkyliques d'acides éthyléniques en $C_4$-$C_5$ présentant un groupe alkyle en $C_1$-$C_8$.

6. Procédé selon la revendication 3, caractérisé en ce qu'un monomère réticulant et/ou un agent limiteur de chaîne est mis en œuvre en proportions comprises entre 0 et 5 % en poids par rapport au(x) monomère(s).

7. Procédé selon la revendication 3, caractérisé en ce que le composé organique insoluble dans l'eau est choisi parmi les hydrocarbures aliphatiques saturés ou non saturés, alicycliques et aromatiques, halogénés ou non, ayant de 10 à 30 atomes de carbone ; les alcools aliphatiques saturés ou non saturés ayant de 10 à 30 atomes de carbone ; les esters d'acides minéraux ou organiques (1-20 C) et d'alcools (1-20 C) dont le nombre d'atomes de carbone est d'au moins 10.

8. Procédé selon la revendication 3, caractérisé en ce que le composé organique représente jusqu'à 50 % en poids de la phase organique contenant la charge magnétique.

9. Procédé selon la revendication 3, caractérisé en ce que la charge magnétique est représentée par des métaux ou leurs alliages avec du molybdène, du chrome, du cuivre, du vanadium, du manganèse, de l'aluminium, du titane ; des oxydes de fers purs, en combinaison ou en mélange avec d'autres oxydes ; du dioxyde de chrome.

10. Procédé selon la revendication 3, caractérisé en ce que la taille des particules de charge magnétique est inférieure à 1 µm.

11. Procédé selon la revendication 3, caractérisé en ce que la quantité de charge magnétique est comprise entre 0,5 et 50 % en poids par rapport au(x) monomère(s).

12. Procédé selon la revendication 3, caractérisé en ce que la phase organique à disperser et homogénéiser avec la phase aqueuse doit être liquide et homogène.

**Revendications** (Pour l'Etat contractant AT)

1. Procédé de préparation de latex de polymères vinylaromatiques hydrophobes magnétiques ayant une concentration inférieure à 65 % en poids en particules de polymères de diamètre moyen compris entre 0,03 et 5 µm, qui renferment 0,5 à 50 % en poids d'une charge magnétique par rapport au polymère, qui consiste à polymériser en milieu aqueux un monomère seul ou en mélange avec un monomère copolymérisable en présence d'une charge magnétique et est caractérisé en ce la charge magnétique est dispersée dans une phase organique formée d'un initiateur organo-soluble, de tout ou partie d'un monomère vinylaromatique seul ou en mélange avec au moins un monomère copolymérisable, donnant un polymère hydrophobe et/ou d'un composé organique insoluble dans l'eau ; la dispersion obtenue est mélangée avec tout ou partie d'une solution aqueuse formée d'eau et d'au moins un agent émulsifiant, puis l'ensemble est homogénéisé pour obtenir des gouttelettes de phase organique de taille comprise entre 0,03 et 5 µm et enfin polymérisé après addition éventuelle du reste des constituants.

2. Procédé selon la revendication 1, caractérisé en ce que les monomères vinylaromatiques, mis en œuvre seuls ou en mélange entre eux, sont insolubles dans l'eau et sont représentés par le styrène, l'alpha-méthylstyrène, l'éthylstyrène, le tertiobutylstyrène, le vinyltoluène.

3. Procédé selon la revendication 1, caractérisé en ce que les monomères copolymérisables sont insolubles dans l'eau et sont choisis parmi les composés diéniques, les acrylates et méthacrylates d'alkyle présentant un groupe alkyle en $C_3$-$C_{10}$, les esters alkyliques d'acides éthyléniques en $C_4$-$C_5$ présentant un groupe alkyle en $C_1$-$C_8$.

4. Procédé selon la revendication 1, caractérisé en ce qu'un monomère réticulant et/ou un agent limiteur de chaîne est mis en œuvre en proportions comprises entre 0 et 5 % en poids par rapport au(x) monomère(s).

5. Procédé selon la revendication 1, caractérisé en ce que le composé organique insoluble dans l'eau est choisi parmi les hydrocarbures aliphatiques saturés ou non saturés, alicycliques et aromatiques, halogénés ou non, ayant de 10 à 30 atomes de carbone ; les alcools aliphatiques saturés ou non saturés ayant de 10 à 30 atomes de carbone ; les esters d'acides minéraux ou organiques (1-20 C) et d'alcools (1-20 C) dont le nombre d'atomes de carbone est d'au moins 10.

6. Procédé selon la revendication 1, caractérisé en ce que le composé organique représente jusqu'à 50 % en poids de la phase organique contenant la charge magnétique.

7. Procédé selon la revendication 1, caractérisé en ce que la charge magnétique est représentée par des métaux ou leurs alliages avec du molybdène, du chrome, du cuivre, du vanadium, du manganèse, de l'aluminium, du titane ; des oxydes de fers purs, en combinaison ou en mélange avec d'autres oxydes ; du dioxyde de chrome.

8. Procédé selon la revendication 1, caractérisé en ce que la taille des particules de charge magnétique est inférieure à 1 µm.

9. Procédé selon la revendication 1, caractérisé en ce que la quantité de charge magnétique est comprise entre 0,5 et 50 % en poids par rapport au(x) monomère(s).

10. Procédé selon la revendication 1, caractérisé en ce que la phase organique à disperser et homogénéiser avec la phase aqueuse doit être liquide et homogène.

**Claims** (for the Contracting States : BE, CH, DE, FR, GB, IT, LI, LU, NL, SE)

1. A latex of magnetic polymers of a concentration in respect of particles of polymers of a mean diameter of between 0.03 and 5 µm, of less than 65 % by weight, characterised in that the particles comprise hydrophobic vinylaromatic polymers and contain from 0.5 to 50 % by weight of a magnetic filler with respect to the polymer.

2. A latex according to claim 1, characterised in that the vinylaromatic polymers are represented by homopolymers of vinylaromatic monomers which are water-insoluble such as styrene, alpha-methyl styrene, ethyl styrene, tertiobutyl styrene and vinyltoluene ; and the copolymers of said monomers with each other and/or with other copolymerisable monomers which are water-insoluble, selected from dienic compounds, alkyl acrylates and methacrylates having a $C_3$-$C_{10}$ alkyl group, and alkyl esters of $C_4$-$C_5$ ethylenic acids having a $C_1$-$C_8$ alkyl group.

3. A process for preparing latexes according to claim 1, which comprises polymerising in an aqueous medium a monomer alone or in mixture with a co-polymerisable monomer in the presence of a magnetic filler and is characterised in that the magnetic filler is dispersed in an organic phase formed by an organo-soluble initiator, all or part of a vinylaromatic monomer alone or in mixture with at least one co-polymerisable monomer, giving a hydrophobic polymer, and/or a water-insoluble organic compound ; the resulting dispersion is mixed with all or part of an aqueous solution formed by water and at least one emulsifying agent, then the whole is homogenised to produce droplets of organic phase of a size of between 0.03 and 5 µm and finally polymerised after the possible addition of the remainder of the constituents.

4. A process according to claim 3, characterised in that the vinylaromatic monomers used alone or in mixture with each other are insoluble in water and are represented by styrene, alpha-methyl styrene, ethyl styrene, tertiobutyl styrene and vinyl toluene.

5. A process according to claim 3, characterised in that the co-polymerisable monomers are insoluble in water and are selected from dienic compounds, alkyl acrylates and methacrylates having a $C_3$-$C_{10}$ alkyl group and alkyl esters of $C_4$-$C_5$ ethylenic acids having a $C_1$-$C_8$ alkyl group.

6. A process according to claim 3, characterised in that a cross-linking monomer and/or a chain limiting agent is used in proportions of between 0 and 5 % by weight with respect to the monomer or monomers.

7. A process according to claim 3, characterised in that the water-insoluble organic compound is selected from halogenated or unhalogenated, alicyclic and aromatic, saturated or unsaturated aliphatic hydrocarbons having from 10 to 30 carbon atoms ; saturated or unsaturated aliphatic alcohols having from 10 to 30 carbon atoms ; and the esters of organic or inorganic acids (1-20 C) and alcohols (1-20 C) in which the number of carbon atoms is at least 10.

8. A process according to claim 3, characterised in that the organic compound represents up to 50 % by weight of the organic phase containing the magnetic filler.

9. A process according to claim 3, characterised in that the magnetic filler is represented by metals or alloys thereof with molybdenum, chromium, copper, vanadium, manganese, aluminium, titanium ; pure iron oxides, in combination or in mixture with other oxides ; and chromium dioxide.

10. A process according to claim 3, characterised in that the size of the particles of magnetic filler is less than 1 μm.

11. A process according to claim 3, characterised in that the amount of magnetic filler is between 0.5 and 50 % by weight with respect to the monomer or monomers.

12. A process according to claim 3, characterised in that the organic phase to be dispersed and homogenised with the aqueous phase must be liquid and homogeneous.


Claims (for the Contracting State AT)

1. A process for preparing latex of magnetic hydrophobic vinylaromatic polymers having a level of concentration of less than 65 % by weight in respect of particles of polymers having a mean diameter of between 0.03 and 5 μm, which contain from 0.5 to 50 % by weight of a magnetic filler with respect to the polymer, which comprises polymerising in an aqueous medium a monomer alone or in mixture with a co-polymerisable monomer in the presence of a magnetic filler and is characterised in that the magnetic filler is dispersed in an organic phase formed by an organo-soluble initiator, all or part of a vinylaromatic monomer alone or in mixture with at least one co-polymerisable monomer, giving a hydrophobic polymer, and/or a water-insoluble organic compound ; the resulting dispersion is mixed with all or part of an aqueous solution formed by water and at least one emulsifying agent, then the whole is homogenised to produce droplets of organic phase of a size of between 0.03 and 5 μm and finally polymerised after the possible addition of the remainder of the constituents.

2. A process according to claim 1, characterised in that the vinylaromatic monomers used alone or in mixture with each other are insoluble in water and are represented by styrene, alpha-methyl styrene, ethyl styrene, tertiobutyl sturene and vinyl toluene.

3. A process according to claim 1, characterised in that the co-polymerisable monomers are insoluble in water and are selected from dienic compounds, alkyl acrylates and methacrylates having a $C_3$-$C_{10}$ alkyl group and alkyl esters of $C_4$-$C_5$ ethylenic acids having a $C_1$-$C_8$ alkyl group.

4. A process according to claim 1, characterised in that a cross-linking monomer and/or a chain limiting agent is used in proportions of between 0 and 5 % by weight with respect to the monomer or monomers.

5. A process according to claim 1, characterised in that the water-insoluble organic compound is selected from halogenated or unhalogenated, alicyclic and aromatic, saturated or unsaturated aliphatic hydrocarbons having from 10 to 30 carbon atoms ; saturated or unsaturated aliphatic alcohols having from 10 to 30 carbon atoms ; and the esters of organic or inorganic acids (1-20 C) and alcohols (1-20 C) in which the number of carbon atoms is at least 10.

6. A process according to claim 1, characterised in that the organic compound represents up to 50 % by weight of the organic phase containing the magnetic filler.

7. A process according to claim 1, characterised in that the magnetic filler is represented by metals or alloys thereof with molybdenum, chromium, copper, vanadium, manganese, aluminium, titanium ; pure iron oxides, in combination or in mixture with other oxides ; and chromium dioxide.

8. A process according to claim 1, characterised in that the size of the particles of magnetic filler is less than 1 μm.

9. A process according to claim 1, characterised in that the amount of magnetic filler is between 0.5 and 50 % by weight with respect to the monomer or monomers.

10. A process according to claim 1, characterised in that the organic phase to be dispersed and homogenised with the aqueous phase must be liquid and homogeneous.


Ansprüche (für die Vertragsstaaten : BE, CH, DE, FR, GB, IT, LI, LU, NL, SE)

1. Latices aus magnetischen Polymeren mit einer Konzentration an Polymerteilchen mit einem mittleren Durchmesser zwischen 0,03 und 5 μm unter 65 Gew.%, dadurch gekennzeichnet, daß die Teilchen aus hydrophoben Vinylaromaten-Polymeren bestehen und 0,5 bis 50 Gew.% einer magnetischen Füllung, bezogen auf das Polymer, enthalten.

2. Latices nach Anspruch 1, dadurch gekennzeichnet, daß die Vinylaromaten-Polymere die Homopolymere aus wasserunlöslichen monomeren Vinylaromaten, wie z. B. Styrol, Alpha-Methylstyrol, Ethylstyrol, Tertiär-Butylstyrol und Vinyltoluol, die Copolymere von diesen Monomeren untereinander und/oder mit anderen wasserunlöslichen copolymerisierbaren Monomeren sind, die aus der Gruppe der

9

**0 038 730**

Dienverbindungen, der Acrylate und Alkylmethacrylate mit einer $C_3$-$C_{10}$-Alkylgruppe, oder der Alkylester der ungesättigten aliphatischen $C_4$-$C_5$-Carbonsäuren mit einer $C_1$-$C_8$-Esteralkylgruppe gewählt sind.

3. Verfahren zur Herstellung der Latices nach Anspruch 1, wobei man ein Monomer allein oder in Mischung mit einem copolymerisierbaren Monomeren in Gegenwart eines magnetischen Füllstoffs in wässrigem Milieu polymerisiert, dadurch gekennzeichnet, daß man den magnetischen Zusatzstoff in einer organischen Phase dispergiert, die aus einem in einem organischen Lösungsmittel löslichen Initiator, der ganzen Menge oder einem Teil des monomeren Vinylaromaten allein oder in Mischung mit mindestens einem copolymerisierbaren Monomeren, die ein hydrophobes Polymer bilden, und/oder einer wasserunlöslichen organischen Verbindung besteht, daß man die erhaltene Dispersion mit der ganzen Menge oder einem Teil einer wässrigen Lösung aus Wasser und wenigstens einem Emulgator mischt, das Ganze homogenisiert, um Tröpfchen organischer Phase in einer Größe zwischen 0,03 und 5 μm zu erhalten und schließlich nach eventueller Zugabe der übrigen Bestandteile polymerisiert.

4. Verfahren nach Anspruch 3, dadurch gekennzeichnet, daß die allein oder in Mischung untereinander verwendeten monomeren Vinylaromaten in Wasser nicht löslich und Styrol, Alpha-Methyl-styrol, Ethylstyrol, Tertiär-Butylstyrol und Vinyltoluol sind.

5. Verfahren nach Anspruch 3, dadurch gekennzeichnet, daß die copolymerisierbaren Monomere in Wasser nicht löslich sind und aus der Gruppe der Dienverbindungen, der Acrylate und Alkylmethacrylate mit einer $C_3$-$C_{10}$-Alkylgruppe oder der Alkylester der ungesättigten aliphatischen $C_4$-$C_5$-Carbonsäuren mit einer $C_1$-$C_8$-Esteralkylgruppe gewählt sind.

6. Verfahren nach Anspruch 3, dadurch gekennzeichnet, daß ein monomeres Vernetzungsmittel und/oder ein kettenlimitierendes Mittel in Mengen zwischen 0 und 5 Gewichtsprozent, bezogen auf das (die) Monomer(e), eingesetzt wird.

7. Verfahren nach Anspruch 3, dadurch gekennzeichnet, daß die wasserunlösliche organische Verbindung aus der Gruppe der gesättigten oder nicht gesättigten aliphatischen, alizyklischen oder aromatischen, halogenierten oder nicht halogenierten Kohlenwasserstoffe mit 10 bis 30 Kohlenstoffatomen, der aliphatischen gesättigten oder ungesättigten Alkohole mit 10 bis 30 Kohlenstoffatomen oder der mindestens 10 Kohlenstoffatome enthaltenden Ester aus mineralischen oder organischen Säuren mit 1 bis 20 Kohlenstoffatomen und Alkoholen mit 1 bis 20 Kohlenstoffatomen gewählt wird.

8. Verfahren nach Anspruch 3, dadurch gekennzeichnet, daß die organischen Verbindung bis 50 Gew.% der die magnetische Füllung enthaltenden organischen Phase ausmacht.

9. Verfahren nach Anspruch 3, dadurch gekennzeichnet, daß die magnetische Füllung aus Metallen oder ihren Legierungen mit Molybdän, Chrom, Kupfer, Vanadium, Mangan, Aluminium, Titan, aus reinen Eisenoxiden, aus Eisenoxiden in Verbindung oder Mischung mit anderen Oxiden, oder aus Chromdioxid besteht.

10. Verfahren nach Anspruch 3, dadurch gekennzeichnet, daß die Teilchengröße des magnetischen Zusatzstoffes unter 1 μm liegt.

11. Verfahren nach Anspruch 3, dadurch gekennzeichnet, daß die Menge der magnetischen Füllung zwischen 0,5 und 50 Gew.%, bezogen auf das (die) Monomer(e), beträgt.

12. Verfahren nach Anspruch 3, dadurch gekennzeichnet, daß die zu dispergierende und mit der wässrigen Phase zu homogenisierende organische Phase flüssig und homogen ist.


**Ansprüche** (für den Vertragsstaat AT)

1. Verfahren zur Herstellung von hydrophoben magnetischen Vinylaromaten-Polymeren mit einer Konzentration an Polymerteilchen mit einem mittleren Durchmesser zwischen 0,03 und 5 μm unter 65 Gew.%, die 0,5 bis 50 Gew.% einer magnetischen Füllung, bezogen auf das Polymer, enthalten, wobei man ein Monomer allein oder in Mischung mit einem copolymerisierbaren Monomeren in gegenwart eines magnetischen Füllstoffes in wässrigem Milieu polymerisiert, dadurch gekennzeichnet, daß man den magnetischen Zusatzstoff in einer organischen Phase dispergiert, die aus einem in einem organischen Lösungsmittel löslichen Initiator, der ganzen Menge oder einem Teil des monomeren Vinylaromaten allein oder in Mischung mit mindestens einem copolymerissierbaren Monomeren, die ein hydrophobes Polymer bilden, und/oder einer wasserunlöslichen organischen Verbindung besteht, die erhaltene Dispersion mit der ganzen Menge oder einem Teil einer wässrigen Lösung aus Wasser und wenigstens einem Emulgator mischt, das Ganze homogenisiert, um Tröpfchen organischer Phase in einer Größe zwischen 0,03 und 5 μm zu erhalten und schließlich nach eventueller Zugabe der übrigen Bestandteile polymerisiert.

2. Verfahren nach Anspruch 1, dadurch gekennzeichnet, daß die allein oder in Mischung untereinander verwendeten monomeren Vinylaromaten in Wasser nicht löslich und Styrol, Alpha-Methylstyrol, Ethylstyrol, Tertiär-Butylstyrol und Vinyltoluol sind.

3. Verfahren nach Anspruch 1, dadurch gekennzeichnet, daß die copolymerisierbaren Monomere in Wasser nicht löslich sind und aus der Gruppe der Dienverbindungen, der Acrylate und Alkylmethacrylate mit einer $C_3$-$C_{10}$-Alkylgruppe oder der Alkylester der ungesättigten aliphatischen $C_4$-$C_5$-Carbonsäuren mit einer $C_1$-$C_8$-Esteralkylgruppe gewählt sind.

4. Verfahren nach Anspruch 1, dadurch gekennzeichnet, daß ein monomeres Vernetzungsmittel

10

und/oder ein kettenlimitierendes Mittel in mengen zwischen 0 und 5 Gewischtsprozent, bezogen auf das (die) Monomer(e), eingesetzt wird.

5. Verfahren nach Anspruch 1, dadurch gekennzeichnet, daß die wasserunlösliche organische Verbindung aus der Gruppe der gesättigten oder nicht gesättigten aliphatischen, alizyklischen und aromatischen, halogenierten oder nicht halogenierten Kohlenwasserstoffe mit 10 bis 30 Kohlenstoffatomen, der gesättigten oder ungesättigten aliphatischen Alkohole mit 10 bis 30 Kohlenstoffatomen oder der mindestens 10 Kohlenstoffatome enthaltenden Ester aus mineralischen oder organischen Säuren mit 1 bis 20 Kohlenstoffatomen und Alkoholen mit 1 bis 20 Kohlenstoffatomen gewählt wird.

6. Verfahren nach Anspruch 1, dadurch gekennzeichnet, daß die organische Verbindung bis 50 Gew.% der die magnetische Füllung enthaltenden organischen Phase ausmacht.

7. Verfahren nach Anspruch 1, dadurch gekennzeichnet, daß die magnetische Füllung aus Metallen oder ihren Legierungen mit Molybdän, Chrom, Kupfer, Vanadium, Mangan, Aluminium, Titan, aus reinen Eisenoxiden, aus Eisenoxiden als Verbindung oder Mischung mit anderen Oxiden, oder aus Chromdioxid besteht.

8. Verfahren nach Anspruch 1, dadurch gekennzeichnet, daß die Teilchengröße des magnetischen Zusatzstoffes unter 1 $\mu$m liegt.

9. Verfahren nach Anspruch 1, dadurch gekennzeichnet, daß die Menge der magnetischen Füllung zwischen 0,5 und 50 Gew.%, bezogen auf das (die) Monomer(e), beträgt.

10. Verfahren nach Anspruch 1, dadurch gekennzeichnet, daß die zu dispergierende und mit der wässrigen Phase zu homogenisierende organische Phase flüssig und homogen ist.